# EUROPEAN PATENT APPLICATION

(11) **EP 2 610 253 A1**
(43) Date of publication of application: **03.07.2013**
(21) Application number: 11195767.6
(22) Date of filing: 27.12.2011
(51) Int. Cl.: C07D 413/12, A61P 31/04, A61P 13/00, A61P 15/02

(54) **Nifuratel sulfoxide for use in the treatment of bacterial infections.**

(71) Applicant: Polichem SA, 1526 Luxembourg (LU)
(72) Inventor: Gagliardi, Stefania, I-20871 Vimercate (MI) (IT); Consonni, Alessandra, I-21012 Cassano Magnago (VA) (IT); Ronzoni, Silvano, I-20822 Seveso (MB) (IT); Bulgheroni, Anna, I-21100 Varese (IT); Ceriani, Daniela, I-21050 Besano (VA) (IT)
(74) Representative: Pistolesi, Roberto

(57) **Abstract**

The present invention is directed to nifuratel sulfoxide. In particular, it is directed to the use of nifuratel sulfoxide, or a physiologically acceptable salt thereof, or a physiologically acceptable cocrystal thereof, to treat bacterial infections and, in particular, to treat infections caused by *Atopobium* and *Gardnerella* species. The invention is further directed to the use of nifuratel sulfoxide to treat bacteriuria, urinary tract infections, infections of external genitalia in both sexes, as well as bacterial vaginosis, or mixed vaginal infections in women, when one or more species of the genera *Atopobium* and *Gardnerella* are among the causative pathogens of those infections.

## Description

The present invention relates to nifuratel sulfoxide or a physiologically acceptable salt thereof or a physiologically acceptable cocrystal thereof. More in details, it relates to the use of nifuratel sulfoxide, or a physiologically acceptable salt thereof, or a physiologically acceptable cocrystal thereof to treat bacterial infections and, in particular, to treat infections caused by *Atopobium* and/or *Gardnerella* species. The invention is further directed to the use of nifuratel sulfoxide to treat bacteriuria, urinary tract infections, infections of external genitalia in both sexes, as well as bacterial vaginosis, or mixed vaginal infections in women caused by one or more species of the genera *Atopobium* and/or *Gardnerella.*

### BACKGROUND OF THE INVENTION

Bacterial vaginosis is considered as a common vaginal disorder in women of reproductive age. Whereas the normal vaginal flora consists of lactobacilli, especially *L. crispatus,* the disturbed vaginal microflora was mainly characterized in the past by the overgrowth of *Gardnerella vaginalis* (formerly known as *Haemophilus vaginalis*) and anaerobic bacteria such as *Mobiluncus* spp., *Mycoplasma hominis* and *Prevotella* spp. *Gardnerella vaginalis* has also been found in urine and in urinary epithelium of male partners of women with bacterial vaginosis, leading to the consideration that bacterial vaginosis is to be included into the sexually transmitted diseases(Swidsinski A et al. Gynecol Obstet Invest 2010, 70:256-63). More recently, the interest for bacterial vaginosis increased because of reports of adverse sequelae of this disorder, such as preterm birth (Hay PE et al. Brit Med J 1994, 308:295-298), pelvic inflammatory disease (Haggerty CL et al. Clin Infect Dis 2004, 39:990-995) and postpartum endometritis (Watts DH et al. Obstet Gynecol 1990, 75:52-58).

The severity of the consequences of those sequelae asks for an adequate treatment of bacterial vaginosis. In the art, the drug of choice in the treatment of bacterial vaginosis is oral or topical metronidazole, a nitroimidazole derivative, which is considered as the golden standard in the management of non mycotic vaginal infections, both for the women and for their male partners. Metronidazole is an antiprotozoarian drug, endowed with therapeutic effect in genital protozoarian infections of both sexes, like trichomoniasis, and also on protozoarian infections of gastrointestinal tract, like intestinal amoebiasis due to *Giardia lamblia.* Metronidazole is also provided of an inhibitory effect on the growth of *Gardnerella vaginalis* and other bacteria, being inactive on the normal flora of lactobacilli.. Nifuratel is a nitrofurane derivative and is considered as the alternative to metronidazole, being endowed of similar effect on protozoa (*Trichomonas* and *Giardia lamblia*) and on *Gardnerella,* with no effect on lactobacilli. Thus, metronidazole and nifuratel are both antiprotozoarian drugs, with an inhibitory effect on *Gardnerella.*

Recently, it has been put in evidence that a new microorganism, named *Atopobium vaginae,* is strongly associated with bacterial vaginosis (Verstraelen H et al. Am J Obstet Gynecol 2004, 191:1130-1132) and is likely to be the main cause of metronidazole treatment failures and of relapses. *Atopobium* is an anaerobe bacteria never described before, a metronidazole resistant organism, that may account for the antimicrobial resistance (up to 30%) associated with the treatment of bacterial vaginosis with metronidazole (Larsson PG et al. APMIS 2005, 113:305-316). *Atopobium vaginae* has been described to constitute a consistent part of the bacteria that form an adherent biofilm on the vaginal epithelium even after standard therapy with metronidazole (Swidsinski A, Mendling W et al. Am J Obstet Gynecol 2008; 198:97.e1-97.e6) and has been found in urine and in urinary epithelium of male partners of women with bacterial vaginosis (Swidsinski A et al. Gynecol Obstet Invest 2010, 70:256-63). WO2010/121980 discloses that nifuratel is provided with an inhibitory effect on the growth of strains of *Atopobium,* that are resistant to metronidazole.

### DESCRIPTION OF THE INVENTION

The object of the present invention is represented by nifuratel sulfoxide or a physiologically acceptable salt thereof, or a physiologically acceptable cocrystal thereof. The structural formula of nifuratel sulfoxide is reported below.

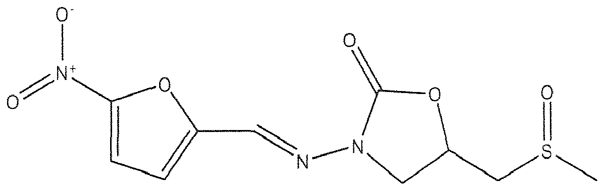

In particular, it is represented by nifuratel sulfoxide or a physiologically acceptable salt thereof, or a physiologically acceptable cocrystal thereof for use in the treatment of bacterial infections and, in particular, in the treatment of any infection caused by *Gardnerella* species or *Atopobium* species.

More in details, the object of the present invention is represented by nifuratel sulfoxide, or a physiologically acceptable salt thereof, or a physiologically acceptable cocrystal thereof, for use in the treatment of bacterial infections in mammalians, preferably in humans, more preferably in women.

According to a preferred embodiment, it is represented by the use of nifuratel sulfoxide, or a physiologically acceptable salt thereof, or a physiologically acceptable cocrystal thereof for treating bacteriuria, urinary tract infections, infections of external genitalia in both sexes, as well as bacterial vaginosis, or mixed vaginal infections in women, when one or more species of the genus *Gardnerella* or *Atopobium* are among the causative pathogens of those infections.

According to an embodiment of the invention, nifuratel sulfoxide may be used in form of a single stereoisomer or a mixture of stereoisomers, wherein said stereoisomers are defined as chemical entities that possess identical constitution, but which differ in the arrangement of their atoms in space. Nifuratel sulfoxide isomers include (R)-cis-nifuratel sulfoxide, (R)-trans-nifuratel sulfoxide, (S)-cis-nifuratel sulfoxide and (S)-trans-nifuratel sulfoxide.

Nifuratel sulfoxide, as used herein, is defined as the mixture of(R)-cis-nifuratel sulfoxide, (R)-trans-nifuratel sulfoxide, (S)-cis-nifuratel sulfoxide and (S)-trans-nifuratel sulfoxide.

(R)-nifuratel sulfoxide, as used herein, is defined as the mixture of (R)-cis-nifuratel sulfoxide and (R)-trans-nifuratel sulfoxide.

(S)-nifuratel sulfoxide, as used herein, is defined as the mixture of (S)-cis-nifuratel sulfoxide and (S)-trans-nifuratel sulfoxide.

"Co-Crystal" as used herein is defined as a crystalline material that comprises two or more compounds; wherein said two or more compounds are in solid state, when in their pure form, at a temperature range from 20°C to 25°C; wherein said two or more compound are present in ionic form, or neutral form, or both; wherein at least one of said two or more compounds is a co-crystal former; wherein at least two of said two or more compounds are held together by weak interaction; wherein said two or more compounds coexist at the molecular level within a single crystal(Zwarotko, Crystal Growth & Design, Vol. 7, No. 1, 2007, 4-9).

"Weak interaction" as used herein is defined as an interaction, at a molecular level, which is neither ionic nor covalent and includes, but it is not limited to: hydrogen bonds, van der Waals forces, and π-π interactions.

"Co-crystal former" as used herein is defined as a molecule being or not being a pharmacological active agent itself and with which nifuratel sulfoxide isomers are able to form co-crystals as previously defined. Examples of co-crystal formers are: antibiotics, antimycotics, anti-inflammatory, hormones and hormone-like molecules.

As a matter of fact, metabolic profiling of nifuratel, following oral administration, was assessed in human urines by liquid chromatography/tandem mass spectrometry (LC/MS/MS).

More in details, urines of two subjects treated orally with nifuratel were collected at the following time-points: pre-dose, 0-4, 4-8, 8-12, 12-24, 24-36 and 36-48 h after treatment.

Nifuratel was not detected in any of the urine samples. However, one major metabolite was found in both subjects and was attributed to sulfoxide nifuratel following the mass spectrometry analysis. Based on such a discovery, the applicant has performed both *in vitro* and *in vivo* tests in order to establish whether nifuratel sulfoxide is endowed with anti-bacterial activity.

Nifuratel sulfoxide was synthesized as racemic mixture and as S-nifuratel sulfoxide and R-nifuratel sulfoxide. The tests *in vitro* have been performed on both the racemic mixture, S-nifuratel sulfoxide and R-nifuratel sulfoxide; the tests *in vivo* have been performed on the racemic mixture only.

### The results of the tests are reported in the examples.

As it shall be appreciated, the *in vitro* antimicrobial activity of nifuratel sulfoxide racemate and of its enantiomers appears to be lower than that of nifuratel, although nifuratel sulfoxide racemate, S-nifuratel sulfoxide and R-nifuratel sulfoxide maintain a good activity against the tested *Gardnerella vaginalis* and *Atopobium vaginae* strains. Nevertheless, nifuratel sulfoxide surprisingly shows a better pharmacokinetic profile in comparison to nifuratel, in particular in terms of clearance, Cₘₐₓ and AUC.

The obtained results demonstrate therefore a far better pharmacokinetic profile of nifuratel sulfoxide, both after intravenous administration and oral gavage, in comparison to nifuratel. Moreover, the higher concentrations achieved in plasma with nifuratel sulfoxide might compensate for the lower *in vitro* antimicrobial activity. Nifuratel sulfoxide, or a physiologically acceptable salt thereof, or a physiologically acceptable cocrystal thereof may thus be used, either in racemic form or in the form of one of its enantiomers, in the treatment of bacterial infections and, in particular, in the treatment of any infection caused by *Gardnerella* or *Atopobium* species.

The physiologically acceptable salt may be selected from, but not limited to: metallic cations (e.g. sodium, potassium, calcium, magnesium, zinc); organic amines (e.g. triethylamine, ethanolamine, triethanolamine, meglumine, ethylene diamine, choline); cationic aminoacids (e.g. arginine, lysine, histidine); inorganic acids (hydrochloride, hydrobromide, phosphate); other organic bases (e.g. procaine, benzathine). The physiologically acceptable cocrystal may be selected from, but not limited to: metallic cations (e.g. sodium, potassium, calcium, magnesium, zinc); organic amines (e.g. triethylamine, ethanolamine, triethanolamine, meglumine, ethylene diamine, choline); aminoacids (e.g. arginine, lysine, histidine, glutamate, aspartate);other organic bases (e.g. procaine, benzathine); inorganic acids (e.g. hydrochloride, hydrobromide, phosphate); sulfonic acids (e.g. mesylate, esylate, isethionate, tosylate, napsilate, besylate)); carboxylic acids (e.g. acetate, propionate, maleate, benzoate, fumarate, salicylate); hydroxyacids (e.g. citrate, lactate, succinate, tartrate, glycolate); fatty acids (e.g. hexanoate, octanoate, decanoate, oleate, stearate).

Nifuratel sulfoxide may be administered to patients by means of the customary pharmaceutical formulations already used for administering nifuratel, such as those described in WO2010/121980, herein incorporated by reference.

The pharmaceutical formulations containing nifuratel sulfoxide, or a physiologically acceptable salt thereof, or a physiologically acceptable cocrystal thereof together with at least a pharmaceutically acceptable excipient and/or adjuvant represent therefore a further object of the present invention.

For instance, solid, semi-solid or liquid formulations of nifuratel sulfoxide or of a physiologically acceptable salt thereof, or a physiologically acceptable cocrystal thereof may be in the form of oral tablets, capsules, dragées or syrup, or topical cream, ointment, gel, lotion, foam, to be applied deeply into the vagina or over the external genitalia, the vulva, the perivulvar area, or the penis, or vaginal tablets, capsules or pessaries, to put deeply into the vagina; such formulations may have a content in nifuratel sulfoxide from 1 to 1000 mg per single dose, more preferably from 10 to 500 mg per single dose, most preferably from 50 to 400 mg per single dose; such formulations may be administered in infected patients according to conventional techniques; according to a preferred embodiment, they are administered on a regular basis, preferably daily.

The pharmaceutical compositions containing nifuratel sulfoxide or a physiologically acceptable salt thereof or a physiologically acceptable cocrystal thereof may be prepared according to conventional techniques, they may contain pharmaceutically acceptable excipients, adjuvants and/or carriers, and they may also contain, in combination, one or more active principles with complementary or, in any case, useful activity.

The active agents which may be used in combination with nifuratel sulfoxide include, but are not limited to, antibiotics, antifungal agents, antiseptic agents, pH modifiers, probiotics; such active ingredients may be administered together with nifuratel sulfoxide (i.e. they may be for instance contained in the same composition as nifuratel sulfoxide) or they may be administered separately from or in temporal proximity with nifuratel sulfoxide.

Examples of antibiotics include clindamycin, macrolide antibiotics such as erythromycin, oleandomycin, flurithromycin, azithromycin and claritromycin and salts thereof, beta-lactam antibiotics such as penicillin, ampicillin, amoxicillin and salts thereof, fluoroquinolones such as ofloxacine, norfloxacine, ciprofloxacine and salts thereof, aminoglycosides such as gentamycin, amikacin, kanamycin, neomycin and salts thereof.

Examples of antifungal agents include: 1-hydroxy-2-pyridone compounds and their salts, e.g. ciclopirox, rilopirox, piroctone, ciclopirox olamine; imidazole derivatives and their salts, e.g. clotrimazole, econazole, isoconazole, ketoconazole, miconazole, tioconazole, bifonazole, fenticonazole and oxiconazole; polyene derivatives and their salts, e.g. nystatin, natamycin and amphotericin; allylamine derivatives and their salts, e.g. naphtifine and terbinafine; triazole derivatives and their salts, e.g. fluconazole, itraconazole, terconazole and voriconazole; morpholine derivatives and their salts, e.g. amorolfine and morpholines disclosed in US-A-5,120,530, herein incorporated by reference; griseofulvin and related compounds, e.g. griseofulvin; undecylenic acid and its salts, in particular, the zinc and calcium salts of undecylenic acid; tolnaphtate and its salts; and flucytosine and its salts.

The antimycotic agent may also be selected from natural sources, in particular plant extracts. Examples of these extracts include tea tree oil (*Melaleuca alternifolia*), lavender oil (*Lavandula officinalis* chaix) and the leaf extract of the neem tree (*Azadirachta indica*).

Examples of the antiseptic agents include: benzalkonium-chlorid, benzethonium-chlorid, cetrimonium-bromid, chlorhexidin, dequaliniumchlorid, triclocarban, triclosan, salicylic acid, benzoic acid and their salts, p-hydroxybenzoic acid and its esters. Examples of pH modifiers include: ascorbic acid, acetic acid, lactic acid, and salts thereof.

Examples of probiotics include species of the genus *Lactobacillus.*

Examples of the compositions prepared according to the present invention include: tablets, capsules, dragées or syrup suitable for oral administration; topical cream, ointment, gel, lotion, foam, to be applied into the vagina or over the external genitalia, the vulva, the perivulvar area, or the penis, the glans, or the balano-preputial skinfold; tablets, capsules or pessaries, to be inserted into the vagina.

### EXAMPLE 1

### The in vitro antimicrobial activity of nifuratel sulfoxide racemate, S-nifuratel sulfoxide and R-nifuratel sulfoxide

The in vitro activity of nifuratel sulfoxide racemate, R-nifuratel sulfoxide and S-nifuratel sulfoxide was tested on a panel of bacteria, which included both Gram positive and Gram negative bacteria.

The minimum inhibitory concentrations (MICs) of the compounds were determined by the broth microdilution method or by the agar dilution method, according to the National Committee for Clinical Laboratory Standards (CLSI) reference methods M07-A8 and M11-A7.

MIC values obtained by the broth microdilution method are reported in table I.

**Table I**

| | µg/ml | | | |
|---|---|---|---|---|
| **Gram positive** | **Nifuratel** | **Nifuratelsulfoxide** | **R-Nifuratelsulfoxide** | **S-Nifuratelsulfoxide** |
| *Micrococcus pyogenes* ATCC 11631 | 1 | 32 | 32 | 64 |
| *Lactobacillus iners*ATCC 55195 | 16 | 16 | 16 | 32 |
| *Staphylococcus aureus* (MRSA) | 2 | 16 | 16 | 16 |
| **Gram negative** | | | | |
| *Escherichia coli* ATCC 11775 | 1 | 16 | 16 | 16 |
| *Proteus mirabilis* ATCC 14153 | 8 | 8 | 8 | 4 |
| *Klebsiella pneumonia* ATCC 10031 | 2 | 16 | 16 | 16 |
| *Shigella sonnei* ATCC 11060 | 1 | 16 | 16 | 16 |
| *Neisseria gonorrhoeae* ATCC 19424 | 0.25 | 2 | 2 | 2 |
| *Salmonellatyphimurium* ATCC 14028 | 4 | 32 | 16 | 16 |
| *Salmonella paratyphiB* ATCC 10719 | 2 | 8 | 16 | 8 |

MIC values obtained by the agar dilution method are reported in table II.

**Table II**

| | **µg/ml** | | | |
|---|---|---|---|---|
| ***Atopobium vaginae*** | **Nifuratel** | **Nifuratelsulfoxide** | **S-Nifuratelsulfoxide** | **R-Nifuratelsulfoxide** |
| CCUG 48515 | 1 | 2 | 2 | 2 |
| CCUG 55226 | 1 | 4 | 8 | 2 |
| CCUG 42099 | 0.5 | 2 | 16 | 1 |
| CCUG 44156 | 0.5 | 1 | 2 | 2 |
| CCUG 39382 | 0.25 | 1 | 2 | 1 |
| CCUG 38953 | 0.125 | 2 | 2 | 1 |
| CCUG 43049 | 0.25 | 4 | 8 | 4 |
| CCUG 55227 | 0.25 | 4 | 4 | 1 |
| CCUG 44061 | 0.5 | 1 | 1 | 1 |

| ***Gardnerella vaginalis*** | **Nifuratel** | **Nifuratelsulfoxide** | **S-Nifuratelsulfoxide** | **R-Nifuratelsulfoxide** |
|---|---|---|---|---|
| ATCC 14018 | 2 | 8 | 16 | 8 |
| C2 | 8 | 16 | 16 | 32 |
| D2 | 4 | 32 | 16 | 64 |
| E2 | 2 | 32 | 32 | 64 |
| G2 | 2 | 32 | 16 | 64 |
| H2 | 1 | 8 | 8 | 16 |
| J2 | 2 | 32 | 16 | 32 |
| K2 | 4 | 32 | 32 | 64 |

Overall, the *in vitro* antimicrobial activity of nifuratel sulfoxide racemate and of its enantiomers was decreased when compared to nifuratel. Nevertheless some activity was retained against all the tested bacteria. In particular, nifuratel sulfoxide racemate. S-nifuratel sulfoxide and R-nifuratel sulfoxide maintained a good activity against the tested *Alopobium vaginae* strains.

### EXAMPLE 2

### Rat pharmacokinetics of nifuratel and nifuratel sulfoxide.

The pharmacokinetic profiles in plasma of nifuratel and nifuratel sulfoxide were tested after intravenous administration (2mg/kg) and oral gavage (15mg/kg) in male rats. Results obtained by the non compartmental analysis (WinNonLin 5.1) of the pharmacokinetic data are reported as means in table III.

**Table III**

| **IV (2mg/Kg)** | **Nifuratel sulfoxide** (n=3) | **Nifuratel** (n=4) |
|---|---|---|
| T₁,₂ (min) | 15.5 | 24 |
| Tₘₐₓ(min) | | 2 2 |
| Cₘₐₓ (ng/ml) | 2221 | 555 |
| C0 (ng/ml) | 2815 | 798 |
| Tₗₐₛₜ (min) | 120 | 120 |
| Cₗₐₛₜ (ng/ml) | 7.3 | 5 |
| AUCₗₐₛₜ (min*ng/ml) | 41988 | 7348 |
| AUC_{inf} (min*ng/ml) | 42159 | 7526 |
| Cl (ml/min/kg) | 47.5 | 267 |
| MRT (min) | 17.7 | 13 |
| Vₛₛ (l/kg) | 0.865 | 4.5 |
| **XOS (15mg/kg)** | **Nifuratel sulfoxide (n=3)** | **Nifuratel (n=4)** |
| T_{1/2} (min) | 128 | 182 |
| Tₘₐₓ (min) | 23 | 30 |
| Cₘₐₓ (ng/ml) | 298 | 8 |
| Tₗₐₛₜ (min) | 240 | 240 |
| Cₗₐₛₜ (ng/ml) | 80.6 | 3 |
| AUCₗₐₛₜ (min*ng/ml) | 36326 | 1218 |
| MRT (min) | 94 | 109 |
| Mean Fpo (%) | 11 | 2.2 |

Nifuratel sulfoxide showed a better pharmacokinetic profile, following 2mg/kg intravenous administration, in comparison to nifuratel, in particular in terms of clearance which was over 5 folds slower for nifuratel sulfoxide with respect to nifuratel. Furthermore, Cₘₐₓ and AUC calculated for nifuratel sulfoxide were respectively 4 and > 5 folds higher than those calculated for nifuratel.

Following 15mg/kg oral administration, this trend was even improved, with Cₘₐₓ and AUC calculated for nifuratel sulfoxide 37 and 30 folds higher than those calculated for nifuratel. This improvement being justified also by a 5 fold increased bioavailability (Mean Fpo 11% for nifuratel sulfoxide versus 2.2% for nifuratel).

### EXAMPLE 3

### Efficiency coefficients of Nifuratel sulfoxide vs. nifuratel

The efficiency coefficient (EC) of nifuratel sulfoxide vs. nifuratel was calculated for each tested strain as follows:
EC = relative oral bioavailability *[MIC(nif)/MIC(nifS)]

Where:
nifS = nifuratel sulfoxide
nif = nifuratel
EC = 1 is indicative of equivalent efficiency between nifuratel sulfoxide and nifuratel.
EC < 1 is indicative of a lower efficiency of nifuratel sulfoxide vs. nifuratel
EC > 1 is indicative of a higher efficiency of nifS vs. nif

The results are reported in tables IV and V.

**Table IV**

| **Gram positive** | **EC** |
|---|---|
| *Micrococcus pyogenes* ATCC 11631 | 0.9 |
| *Stapnylococcus aureus* (MRSA) | 3.8 |
| **Gram negative** | |
| *Escherichia coli* ATCC 11775 | 1.9 |
| *Proteus mirabilis* ATCC 14153 | 30 |
| *Klebsiella pneumonia* ATCC 10031 | 3.8 |
| *Shigella sonnel* ATCC 11060 | 1.9 |
| *Neisseria gonorrhoeae* ATCC 19424 | 3.8 |
| *Salmonella typhimurium* ATCC 14028 | 3.8 |
| *Salmonella paratyphi B* ATCC 10719 | 7.5 |

As reported in table IV, the efficiency coefficient was in favour of nifuratel sulfoxide in all tested strains but one.

**Table V**

| ***Atopobium vaginae*** | **EC** |
|---|---|
| CCUG 48515 | 15 |
| CCUG 55226 | 7.5 |
| CCUG 42099 | 7.5 |
| CCUG 44156 | 15 |
| CCUG 39382 | 7.5 |
| CCUG 38953 | 1.9 |
| CCUG 43049 | 1.9 |
| CCUG 55227 | 1.9 |
| CCUG 44061 | 7.5 |
| ***Gardnerella vaginalis*** | |
| ATCC 14018 | 7.5 |
| C2 | 15 |
| D2 | 3.8 |
| E2 | 1.9 |
| G2 | 1.9 |
| H2 | 3.8 |
| J2 | 1.9 |
| K2 | 3.8 |

As reported in table V, the efficiency coefficient was in favour of nifuratel sulfoxide in all *Atopobium* and *Gardnerella* tested strains.

## Claims

1. Nifuratel sulfoxide or a physiologically acceptable salt thereof or a physiologically acceptable cocrystal thereof.

2. Nifuratel sulfoxide, **characterized by** being in form of a single stereoisomer or a mixture of stereoisomers.

3. Nifuratel sulfoxide according to claims 1 or 2, for use in the treatment of any bacterial infection.

4. Nifuratel sulfoxide for use according to claim 3, **characterized in that** said bacterial infection is caused by one or more species of the genus *Atopobium.*

5. Nifuratel sulfoxide for use according to claim 4, **characterized in that** said species is *Atopobium vaginae.*

6. Nifuratel sulfoxide for use according to claim 3, **characterized in that** said infection is selected from bacteriuria, urethritis, urinary infections or infections of external genitalia in men and/or women.

7. Nifuratel sulfoxide for use according to claim 3, **characterized in that** said infection is selected from bacterial vaginosis or mixed vaginal infections in women.

8. Nifuratel sulfoxide for use according to claim 3, **characterized in that** it is administered to a mammalian, preferably to a human, more preferably to a woman.

9. Nifuratel sulfoxide for use according to claim 8, **characterized in that** it is administered on a daily basis.

10. Nifuratel sulfoxide for use according to claim 8, **characterized in that** it is administered at a dosage from 1 to 1000 mg per single dose, preferably from 10 to 500 mg per single dose, more preferably from 50 to 400 mg per single dose.

11. Nifuratel sulfoxide for use according to claim 8, **characterized in that** it is administered in combination or in temporal proximity with at least one active principle selected from antibiotics, antifungal agents, antiseptic agents, pH modifiers, probiotics.

12. A pharmaceutical formulation containing nifuratel sulfoxide, or a physiologically acceptable salt thereof, or a physiologically acceptable cocrystal thereof together with at least a pharmaceutically acceptable excipient and/or adjuvant.

13. A pharmaceutical formulation according to claim 12, **characterized in that** nifuratel sulfoxide is in form of a single stereoisomer or a mixture of stereoisomers.

14. A pharmaceutical formulation according to claims 12 or 13, **characterized in that** it is in the form of tablet, capsule, dragée or syrup suitable for oral administration; topical cream, ointment, gel, lotion or foam, to be applied into the vagina or over the external genitalia, the vulva, the perivulvar area, or the penis, the glans, or the balano-preputial skinfold; tablet, capsule or pessary to be inserted into the vagina.

15. A pharmaceutical formulation according to claims 12 or 13, **characterized in that** said formulation has a content in nifuratel sulfoxide or a salt thereof, or a physiologically acceptable cocrystal thereof from 1 to 1000 mg, preferably from 10 to 500 mg, more preferably from 50 to 400 mg.

16. A pharmaceutical formulation according to claims 12 or 13, **characterized by** containing a further active principle.

17. A pharmaceutical formulation according to claims 12 or 13, **characterized in that** said active principle is selected from antibiotics, antifungal agents, antiseptic agents, pH modifiers or probiotics.
